(19) Europäisches Patentamt
European Patent Office
Office européen des brevets

(11) Veröffentlichungsnummer: **0 098 430**
**B1**

(12) # EUROPÄISCHE PATENTSCHRIFT

(45) Veröffentlichungstag der Patentschrift: **29.08.90**

(21) Anmeldenummer: **83105956.3**

(22) Anmeldetag: **18.06.83**

(51) Int. Cl.[5]: **C 07 D 417/12,**
C 07 D 251/54,
C 09 B 29/045, C 09 B 29/30,
C 09 B 29/36, C 09 B 35/03,
C 09 B 62/085, C 09 B 62/09,
D 21 H 21/28

(54) **Triazinverbindungen, ihre Herstellung und ihre Verwendung.**

(30) Priorität: **02.07.82 DE 3224786**

(43) Veröffentlichungstag der Anmeldung:
**18.01.84 Patentblatt 84/03**

(45) Bekanntmachung des Hinweises auf die Patenterteilung:
**29.08.90 Patentblatt 90/35**

(84) Benannte Vertragsstaaten:
**CH DE FR GB LI**

(56) Entgegenhaltungen:
**EP-A-0 036 838**
**EP-A-0 062 825**
**EP-A-0 065 595**
**EP-A-0 073 178**
**DE-A-2 711 150**
**DE-A-2 838 271**
**FR-A-2 525 620**

(73) Patentinhaber: **BAYER AG**
**D-5090 Leverkusen 1 Bayerwerk (DE)**

(72) Erfinder: **Stöhr, Frank-Michael, Dr.**
**Weidenweg 25**
**D-5093 Burscheid (DE)**
Erfinder: **Nickel, Horst, Dr.**
**Fontanestrasse 23**
**D-5090 Leverkusen (DE)**

**EP 0 098 430 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung sind Triazinverbindungen der Formel

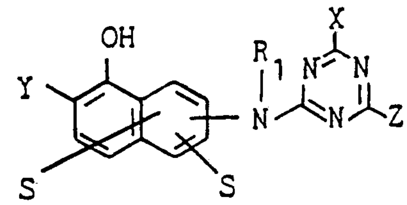

(I)

worin

$R_1$ Wasserstoff oder Alkyl,

S eine Sulfonsäuregruppe oder deren Salz,

X Hydroxy, Alkoxy, Alkyl, Aryl oder eine gegebenenfalls mono- oder di-substituierte Aminogruppe,

Y Wasserstoff oder den Rest eines Azofarbstoffs,

Z ein Rest der Formel

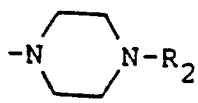 oder 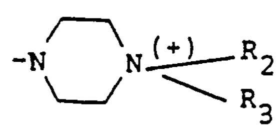 $A^{(-)}$,

$R_2$ und $R_3$ unabhängig voneinander Wasserstoff, Alkyl, Alkenyl oder Aralkyl und

$A^{\ominus}$ ein Anion bedeuten,

worin die cyclischen und acyclischen Reste weitere Substituenten tragen können, und worin im Farbstoffmolekül die Summe der basischen und/oder kationischen Gruppen größer als 2 ist, und das Molekül mindestens eine basische oder Kationische gruppe mehr enthält als das Molekül Sulfonsäuregruppen besitzt, mit Ausnahme des Farbstoffe des Bep. 3i des FR—A 2 525 620, ihre Herstellung und — wenn Y für den Rest eines Azofarbstoffs steht — ihre Verwendung zum Färben von synthetischen und natürlichen Materialien, insbesondere Papier, und — wenn Y für Wasserstoff steht — ihre Verwendung zur Herstellung von Azofarbstoffen.

Die Substituenten S liegen je nach pH-Wert als Sulfonsäure, als Betain oder als sulfonsaures Salz, z.B. als Na-, K-, Li-, Ammonium- oder Mono-, Di-, Tri-hydroxyethyl-ammonium-Salz vor.

Bevorzugt sind Verbindungen der Formel (I), worin die Substituenten S in 3,5 oder 3,6-Stellung, der Aminotriazinylrest in 8-Stellung (OH in 1-Stellung) und $R_1$ für Wasserstoff stehen.

Hervorzuheben sind Azofarbstoffe der Formeln

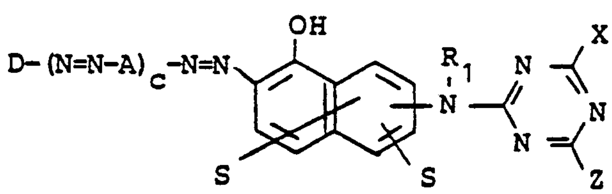

(II)

worin

D den Rest einer Diazokomponente der Benzol-, Naphthalin- oder heterocyclischen Reihe,

A den Rest einer Kupplungskomponente der Benzol- oder Naphthalinreihe und

c 0 oder 1 bedeuten, und

S, X, Z und $R_1$ die Bedeutung der Formel (I) haben,

und worin im Farbstoffmolekül die Summe der basischen und/oder kationischen Gruppen mindestens 3 ist, und

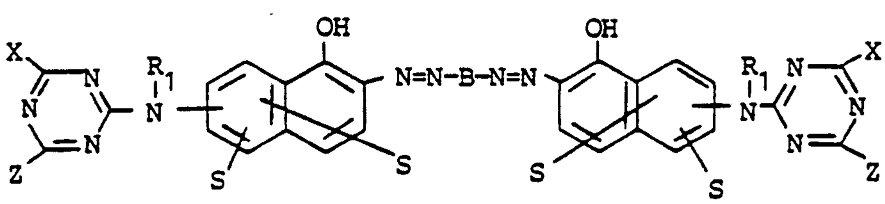

(III)

worin

B den Rest einer aromatischen Tetrazokomponente darstellt,

S, X, Z und $R_1$ die Bedeutung der Formel (I) haben,

und worin die Summe der basischen und/oder kationischen Gruppen im Molekül größer als 4, vorzugsweise größer als 5 ist.

Die Anzahl der basischen und kationischen Gruppen ist von entscheidender Bedeutung für die Wasserlöslichkeit der Farbstoffe, da die Sulfonsäuregruppen mit den basischen oder kationischen Gruppen innere, schwerlösliche Salze bilden können und erst überzählige basische Gruppen in Form der Säureadditionssalze und/oder kationische Gruppen die Wasserlöslichkeit bewirken.

Dabei kann (können) die zusätzliche(n) basische(n) und/oder kationische(n) Gruppe(n) sowohl in A, D und B als auch in X lokalisiert sein, sowie als Substituenten der Reste $R_2$ bzw. $R_3$ auftreten.

Unter einem Alkylrest wird vorzugsweise $C_1$—$C_4$-Alkyl und unter einem Alkoxyrest vorzugsweise $C_1$—$C_4$-Alkoxy verstanden.

Ein Alkenylrest hat vorzugsweise 3 oder 4 C-Atome.

Aryl steht insbesondere für Phenyl oder Naphthyl und Aralkyl für Benzyl oder Phenylethyl.

Als weitere Substituenten dieser Reste kommen vorzugsweise Halogen, Hydroxy, $C_1$—$C_4$-Alkoxy, Cyan, Amino oder Mono- und Di-$C_1$—$C_4$-alkylamino respektive Ammonium und für die aromatischen Reste zusätzlich $C_1$—$C_4$-Alkyl in Frage.

X steht bevorzugt für eine mono- oder disubstitiuerte Aminogruppe oder $NH_2$.

Von den Farbstoffen der Formeln (II) und (III) sind die der Formeln

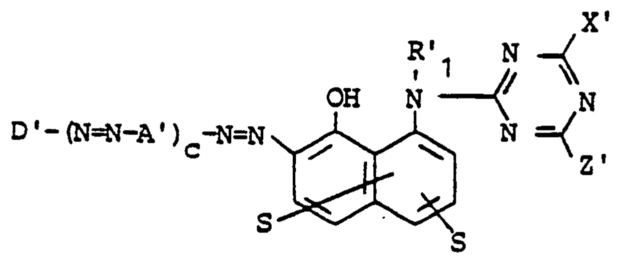

(IV)

und

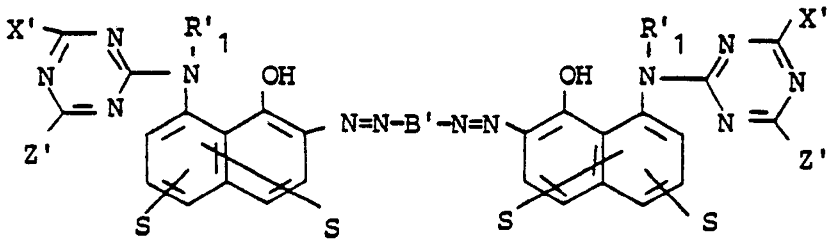

(V)

bevorzugt, worin

$R'_1$ Wasserstoff oder Methyl,

X' einen Rest der Formel

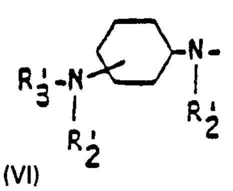 , 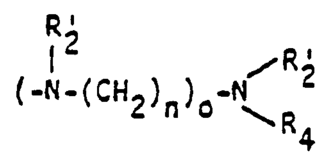

(VI) (VII)

oder 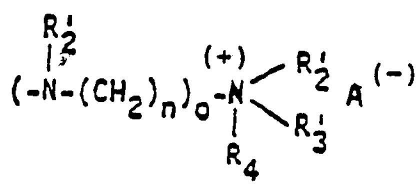 (VIII)

Z' einen Rest der Formel

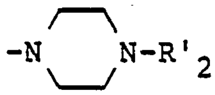 oder 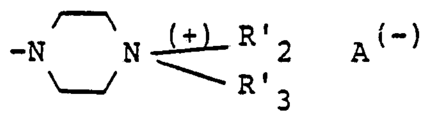

$R'_2$ und $R'_3$ unabhängig voneinander Waserstoff, $C_1$—$C_4$-Alkyl, $C_3$- oder $C_4$-Alkenyl, Benzyl, Phenylethyl, die durch Hydroxy, $C_1$—$C_4$-Alkoxy, Halogen, Cyan, Amino oder Mono- und Di-$C_1$—$C_4$-alkylamino respektive Ammonium und der Benzyl- und Phenylethylrest zusätzlich durch $C_1$- bis $C_4$-Alkyl substituiert sein können,

n 2 oder 3 und zusätzlich 0, wenn o = 1 ist,

o 0, 1 oder 2,

$R_4$ den Rest $R'_2$ und zusätzlich gegebenenfalls durch Halogen, $C_1$—$C_4$-Alkyl, Hydroxy, $C_1$—$C_4$-Alkoxy und/oder den Rest

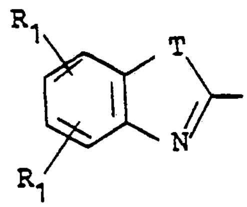 T = S, N-H (IX)

substituiertes Phenyl oder Naphthyl, Benzthiazolyl-(2) oder Isobenzthiazolyl-(3) bedeuten, oder

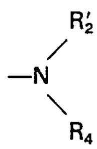

einen Pyrrolidin-, Piperidin- oder Morpholinring oder einen Ring der Formel

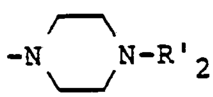 oder 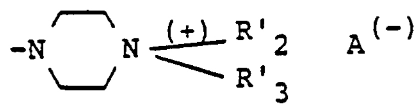

bedeutet,

D' einen Phenyl-, Naphthyl-, Benzthiazolyl- oder Dibenzofuranylrest,

A' einen Phenylen- oder Naphthylenrest,

B' einen Phenylen- oder Naphthylenrest oder einen Rest der Formel

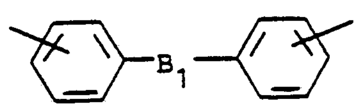

bedeuten, worin

$B_1$ eine direkte Bindung, —$(CH_2)_p$, —NH—CO—, —O—, —$SO_2$—, —NHCONH—, —O—$(CH_2)_p$—O—, —NH—CO—$(CH_2)_p$—CO—NH—, —CO—NH—$(CH_2)_p$—NH—CO— oder

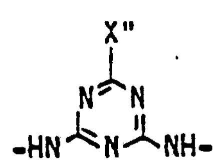

p 2 oder 3 bedeuten, und worin

D', A' und B' durch Halogen, $C_1$—$C_4$-Alkyl, $C_1$—$C_4$-Alkoxy, Acetylamino, Benzoylamino, Phenoxy oder die Gruppen (VII) bis (IX),

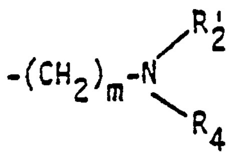 , 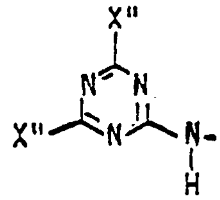 , 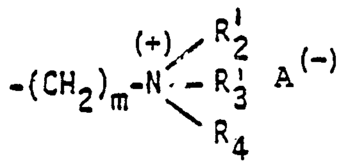

oder eine Sulfonsäuregruppe substituiert sein können,

X'' den Rest X' und zusätzlich die Reste

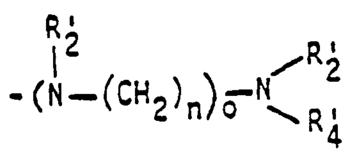 oder 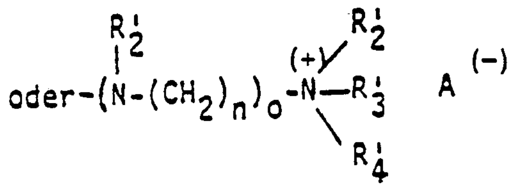

bedeuten, worin

$R_4'$ gegebenenfalls durch die Reste

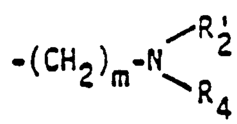 oder 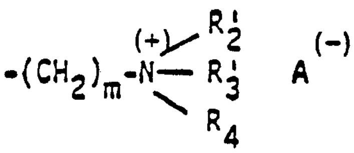

substituiertes Phenyl oder Naphthyl bedeutet,

m und c 0 oder 1 sein können, und

S und $A^{\ominus}$ die Bedeutung der Formel (I) haben.

Die Auswahl der Diazo- bzw. Tetrazokomponenten muß in der Weise erfolgen, daß die Bedingung der Wasserlöslichkeit erfüllt wird, d.h., daß das entstehende Farbsmolekül mindestens eine basische oder kationische Gruppe mehr enthält, als das Molekül Sulfonsäuregruppen besitzt.

Besitzt also die Kupplungskomponente (I) (Y = Wasserstoff) in X und/oder Z drei oder mehr basische bzw. kationische Gruppen, so kann eine beliebige aromatische, carbocyclische oder heterocyclische Diazo- bzw. Tetraazokomponente — die frei ist von anionischen Gruppen — wie beispielsweise Anilin, Aminoazobenzol, Aminonaphthalin, Dehydrothiotoluidin, 4,4'-Diamino-benzoylanilid, 4,4'-Diamino-3,3'-dimethyl- bzw. -dimethoxydiphenyl, 4,4'-Diaminodiphenyl-ethan (1, 2) Verwendung finden.

Besitzt die Kupplungskomponente (I) (Y = Wasserstoff) dagegen nur ein oder zwei basische oder kationische Gruppen, so muß die aromatische carbocyclische oder heterocyclische Diazo- bzw. Tetrazokomkponente mindestens eine bzw. mindestens zwei basische oder kationische Gruppen aufweisen, um in der Salzform der basischen Gruppen oder der kationischen Gruppen die Wasserlöslichkeit zu erreichen.

Geeignete Diazokomponenten dieser Art sind beispielsweise Anilin-3- oder -4-trimethylammoniumchlorid, -methosulfat, -sulfat, -benzolsulfat, -tosylat, 3- oder 4-Aminobenzylidi- oder -trimethylammoniumchlorid, -methosulfat oder 2-Aminonaphthalin-5-methylentrimethylammoniummethosulfat.

Hierzu zählen auch Aminoazoverbindungen mit gegebenenfalls über eine Methylengruppe gebundenen, gegebenenfalls alkylierten Amino- oder Ammoniumgruppen, wie beispielsweise

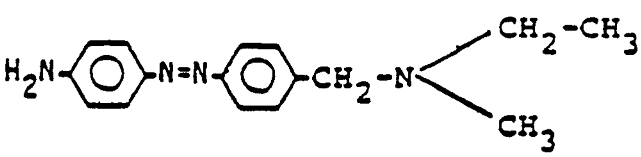

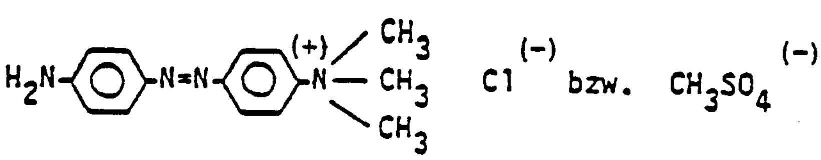

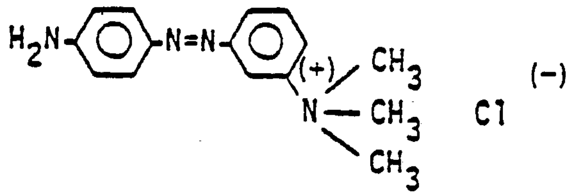

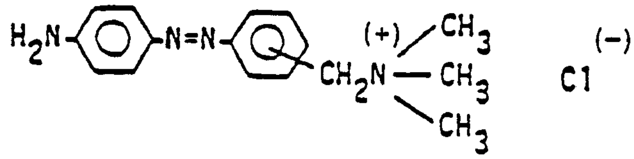

ferner Verbindungen der obengenannten Art, bei denen der Benzolkern durch Methyl, Methoxy, Ethoxy oder Chlor noch weiter substituiert ist, sowie die Kupplungsprodukte von 2-Aminonaphthalin-5-methylentrimethylammoniumchlorid → Anilin bzw. → 3-Methylanilin bzw. → 2-Methoxyanilin bzw. → 2-Methoxy-5-methylanilin.

Als Anion $A^{\ominus}$ kommen übliche farblose organische und anorganische Anionen, beispielsweise Chlorid, Bromid, Jodid, Hydroxyl, Hydrogensulfat, Sulfat, Nitrat, Dihydrogenphosphat, Hydrogenphosphat, Carbonat, Methosulfat, Ethosulfat, Formiat, Acetat, Propionat, Benzolsulfonat und Toluolsulfonat in Betracht.

Das Anion ist im allgemeinen durch das Herstellungsverfahren gegeben. Die Anionen können in bekannter Weise gegen andere Anionen ausgetauscht werden. Vorzugsweise liegen die Chloride, Hydrogensulfate, Sulfate, Methosulfate, Phosphate, Formiate, Acetate oder Methylsulfonate vor.

Die Kupplungskomponenten (I) (Y = H) werden in bekannter Weise im allgemeinen folgendermaßen hergestellt:

Die Naphthalindisulfonsäure (X), vorzugsweise die 3,6-Disulfonsäure, wird zunächst mit Cyanurhalogenid, bevorzugt -chlorid oder -fluorid, zur 1:1-Verbindung umgesetzt. Anschließend wird durch halogenaustausch, beispielsweise mit Ammoniak oder Aminen HX der X-Substituent eingeführt.

Nach Einführung des Substituenten X in der zweiten Stufe bei vorzugsweise 20—50°C wird in dritter Stufe bei 70—95°C die Kondensation mit der Aminoverbindung

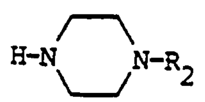

(XI)

oder

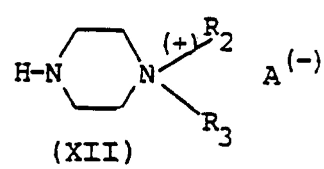

(XII)

vorgenommen.

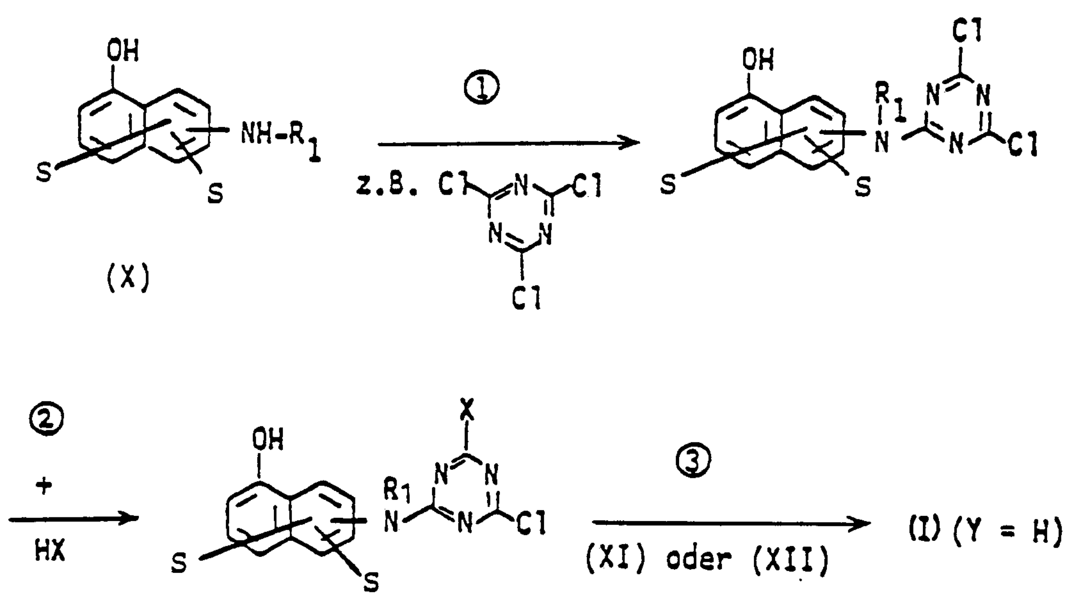

Selbstverständlich können die einzelnen Kondensationsschritte vertauscht und beispielsweise zuerst die Verbindungen (XI) oder (XII) mit dem Cyanurhalogenid kondensiert bzw. zuerst der Substituent Z und zuletzt der Substituent X eingeführt werden.

Bevorzugte Verbindungen (XI) sind substituiertes Piperazin, N-Methyl- und N-(2-Hydroxyethyl)-piperazin sowie besonders bevorzugt N-(2-Aminoethyl)piperazin und N-(3-Dimethylaminopropyl)-piperazin.

Als Amine HX seien folgende genannt:

$C_1$—$C_4$-Mono- bzw. Dialkylamine wie Methyl-, Ethyl-, Chlorethyl-, n-Propyl-, iso-Propyl-, Butyl-, Hydroxy-ethyl-, 2-Methoxy-ethyl-, Dimethyl-, Diethyl-, Di-i-propyl-, Di-n-propyl-, Diethanol-, Di-isopropanol-, Di-iso-butyl-, Methyl-ethyl-, Methyl-ethanol- und Ethyl-ethanolamin, 3-Methylaminopropionitril, Cycloalkylamine wie Pyrrolidin, Piperidin, Morpholin, Piperazin, N-Methyl-piperazin, N-(2-Hydroxy-ethyl)-piperazin, N-(2-Amino-ethyl)-piperazin, N-(3-Dimethylaminopropyl)-piperazin oder entsprechende Piperazinium-Salze; Aralkylamine wie Benzylamin, Benzylmethylamin, Benzylethanolamin, die im Phenylkern durch Chlor oder Methyl substituiert sein können, Diamine wie Ethylendiamin, N,N-Dimethylethylendiamin, 1-Amino-2-diethylaminoethan, Propylendiamin, N-Methyl- und N,N-Dimethyl- oder -ethylpropylendiamin, Dipropylentriamin, Diethylentriamin, N,N',N''-Trimethyldiethylentriamin, 1,4-Diaminocyclohexan, aromatische Amine wie Anilin, N-Methyl oder Ethyl- oder Hydroxyethylanilin, die im Phenylkern in o-, m- oder p-Stellung durch Methyl, Ethyl, Chlor, Methoxy oder Ethoxy substituiert sein können, 1-Naphthylamin, Dehydrothiotoluidin oder -Xylidin, 2-Amino-benzthiazol, 3-Aminoisobenzthiazol, ferner Methyl- und Dimethylhydrazin.

Als Amine HX seien zusätzlich folgende genannt:

Aromatische Amine wie Anilin, N-Methyl- oder Ethyl- oder Hydroxyethylanilin, die im Phenylkern in m- oder p-Stellung durch N,N-Dimethyl- oder N,N-Diethylamino, Trimethylammoniumchlorid, -methosulfat, -acetat oder -tosylat, Methylendimethyl-, -diethylamino oder Methylentrimethylammoniumchlorid substituiert sind, oder 2-Aminonaphthalin-5-methyltrimethylammoniumchlorid.

Die neuen Azofarbstoffe (I) (Y = Rest eines Azofarbstoffs) werden nach üblichen, in der Azochemie bekannten Verfahren hergestellt (siehe z.B. Houben-Weyl, Methoden der organischen Chemie, Band X/3, Georg Thieme-Verlag, Stuttgart, 1965 ab Seite 226 und Seite 270) durch Kupplung von Diazonium- bzw. Tetrazoniumverbindungen mit Kupplungskomponenten der Formel (I) (Y = H), vorzugsweise in wäßrigem Medium. Falls X = Alkyl oder Aryl, sind natürlich nur 2 austauschbare Halogen-Substituenten im Triazinring zur Verfügung, wobei wahlweise zuerst (XI) bzw. (XII) oder die Aminonaphtholdisulfonsäure (X) kondensiert werden kann.

Die Farbstoffe werden zum Färben von mit kationischen Farbstoffen färbbaren Materialien verwendet. Genannt seien beispielsweise: Polyacrylnitril, sauer modifizierte Polyester, z.B. Polyglykolterephthalate, wie sie in der belgischen Patentschrift 549 179 oder US-Patentschrift 2 893 816, beschrieben werden, sauer modifizierte Polyamide, tannierte vegitabilische Fasern (Baumwolle), Leder und vorzugsweise Papier. Geeignet sind die Farbstoffe zum Färben von geleimten sowie ungeleimtem Papier, wobei von gebleichtem oder ungebleichtem Zellstoff ausgegangen werden kann und Laub- oder Nadelholz-Zellstoff wie Birken- und/oder Kiefernsulfid und/oder Sulfat-Zellstoff verwendet werden kann.

Die Farbstoffe werden sowohl als Pulver- bzw. Granulat-Präparationen als auch in Form von konzentrierten Lösungen zum Einsatz gebracht. Pulver-Präparationen werden in üblicher Weise mit Stellmaterialien wie Natriumsulfat, -phosphat, -chlorid, -acetat in Gegenwart von Entstaubungsmitteln eingestellt, oder die Farbstoffe werden direkt als Sprühtrockungspräparationen in den Handel gebracht. Konzentreierte Farbstofflösungen können wäßriger oder wäßrig/organischer Art sein, wobei übliche umweltfreundliche und möglichst gut abbaubare Zusätze bevorzugt werden, wie anorganische oder organische Säuren, vorzugsweise Essigsäure Hydroxyessigsäure, Ameisensäure, Milchsäure, Zitronensäure, Methansulfonsäure, Phosphorsäure, Amide wie Formamid, Dimethylformamid, Harnstoff, N,N'-Dimethyl-Harnstoff, Alkohole wie Glykol, Diglykol, Diglykolether, vorzugsweise Methyl-, Ethyl- oder Butylether sowie Hydroxypropionitril und Caprolactam.

Die Farbstoffe besitzen ein ausgezeichnetes Ziehvermögen und sehr gute Allgemein-Echtheiten. Papierfärb ungen zeichnen sich durch sehr gute Naßechtheiten sowie Alaun-, Säure- und Alkali-Echtheiten aus. Sie besitzen eine überraschend hohe Lichtechtheit bei gleichzeitig hoher Klarheit und Farbstärke.

In der EP—A 0 036 838 werden Azofarbstoffe beschrieben, welche einen Halogentriazinylaminorest an eine Oxynaphthalinsulfonsäure gebunden enthalten und in der DE—A 27 11 150 Azofarbstoffe mit Fluorotriazinylaminorest gebunden an eine Oxynaphthalinsulfonsäure-Kupplungskomponente. In der älteren FR—A 25 25 620 wird in Beispiel 31 der Farbstoff der Formel

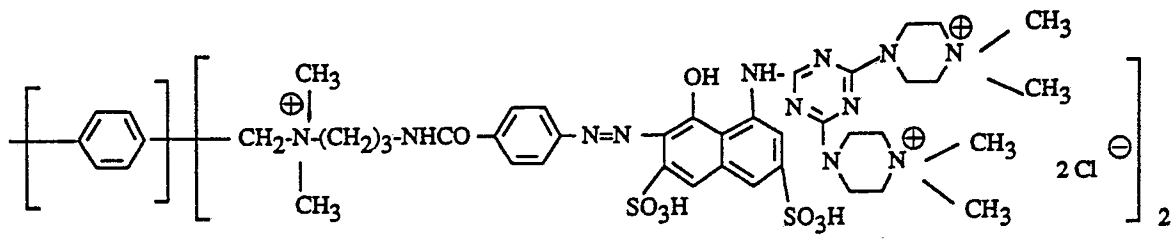

beschrieben.

Beispiel 1

242 g (0,761 Mol) frisch gefällte 4-Amino-5-hydroxy-2,7-naphthalindisulfonsäure wird bei 0—5°C mit 142 g (0,769 Mol) Cyanurchlorid umgesetzt. Der pH-Wert wird mit 10%iger Natronlauge 1—1,5 Stunden bei 3,5 gehalten. Danach stürzt man 206 g (1,595 Mol) N-2-Aminoethylpiperazin ein. Die Temperatur steigt auf 20—25°C und der pH-Wert auf 9,5—10 an. Anschließend wird auf 90°C aufgeheizt und der pH-Wert mit 10%iger Natronlauge bei 10 gehalten. Nach dem Abkühlen auf Raumtemperatur stellt man mit 28%iger Salzsäure pH 4—5 ein. Zu dieser Lösung gibt man die Diazotierung von 174 g (0,725 Mol) 4-(6-Methylbenzthiazo-2-yl)anilin. Der pH wird mit festem Natriumhydrogencarbonat langsam auf 6—7 gestellt. Nach ca. 1,5-Stunden wird der ausgefallene Farbstoff der Formel

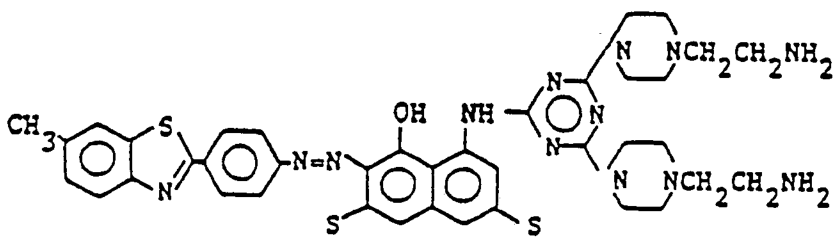

bzw. dessen Hydrochlorid abgesaugt und mit Wasser gewaschen. Die Paste wird in Wasser, Ameisensäure und Methansulfonsäure gelöst, und man erhält eine konzentrierte, stabile und gebrauchsfertige Farbstofflösung, die Papier in blaustichig violetten Tönnen färbt.

Beispiel 2

Verwendet man statt der in Beispiel 1 eingesetzten Diazokomponente eine äquivalente Menge 4,4'-Diaminobenzoylanilid und verfährt dann weiter wie in Beispiel 1 angegeben, erhält man eine konzentrierte, stabile und gebrauchsfertige Farbstofflösung des Farbstoffs der Formel

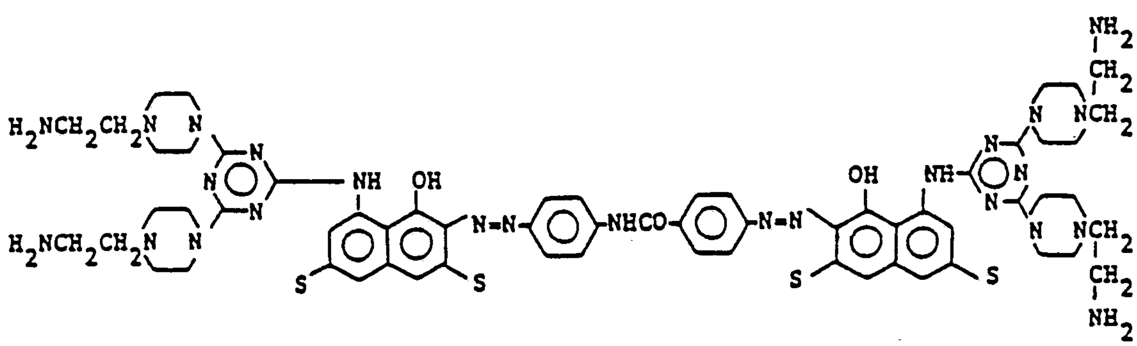

bzw. des Hydrochlorides dieses Farbstoffs, der Papier in blaustichig violetten Tönen färbt.

Ersetzt man in Beispiel 1 4-(6-Methylbenzthiazo-2-yl)anilin durch die in der Tabelle genannten Diazokomponenten erhält man Farbstoffe, die Papier in den angegebenen Farbtönen färben.

| Bsp. | Diazokomponente | Farbton |
|---|---|---|
| 3 | 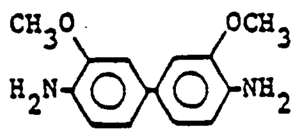 | rotstichig blau |
| 4 | 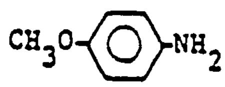 | violett |
| 5 | 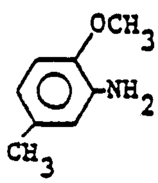 | violett |
| 6 | 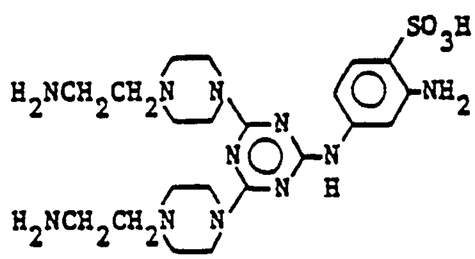 | blaustichig pink |
| 7 | 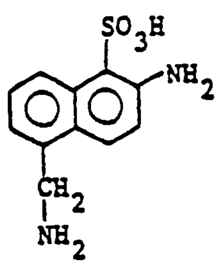 | blaustichig pink |
| 8 | 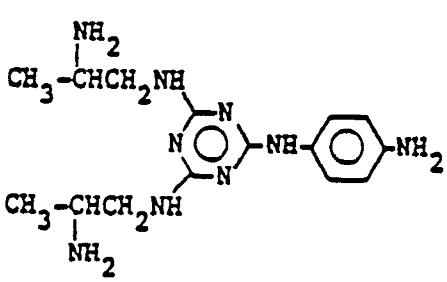 | blaustichig violett |

| Bsp. | Diazokomponente | Farbton |
|---|---|---|
| 9 | 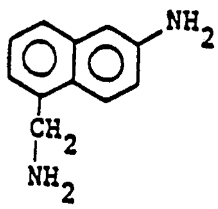 | rotstichig violett |
| 10 | 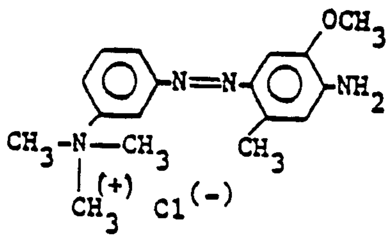 | rotstichig marineblau |

Beispiel 11

Verwendet man statt der in Beispiel 1 eingesetzten 4-Amino-5-hydroxy-2,7-naphthalindisulfonsäure eine äquimolare Menge 4-Amino-5-hydroxy-1,7-naphthalindisulfonsäure und verfährt dann weiter wie in Beispiel 1 angegeben, erhält man einen Farbstoff der Formel

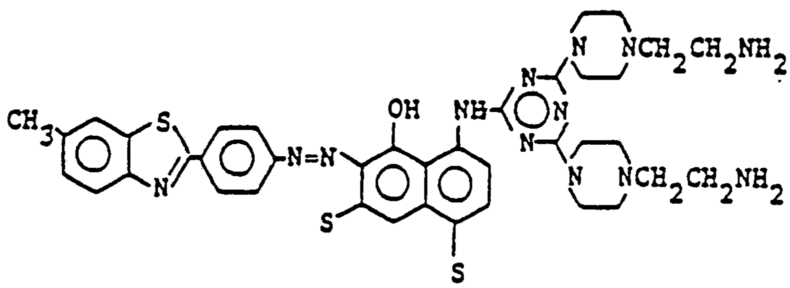

bzw. das Hydrochlorid dieses Farbstoffs, der Papier in blaustichig violetten Tönen färbt.

Ersetzt man in Beispiel 1 4-(6-Methylbenzthiazo-2-yl)anilin durch die in der Tabelle genannten Diazokomponenten und die Kupplungskomponente durch die des Beispiels 11, so erhält man Farbstoffe, die Papier in den angegebenen Farbtönen färben.

| Bsp. | Diazokomponente | Farbton |
|---|---|---|
| 12 | 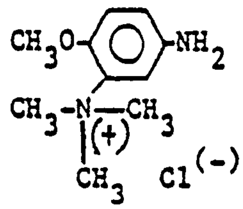 | blaustichig pink |
| 13 | 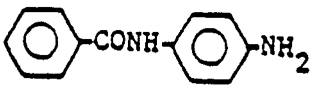 | violett |
| 14 | 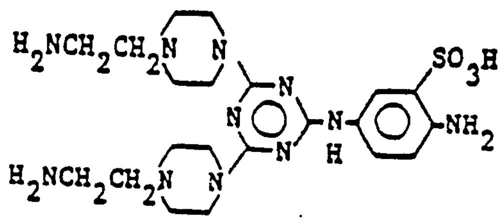 | blaustichig violett |
| 15 | 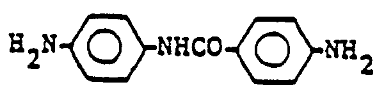 | blaustichig violett |

Beispiel 16

Ersetzt man bei der Herstellung der Kupplungskomponenten das in den vorstehenden Beispielen benutzte N-2-Aminoethylpiperazin komplett oder teilweise (ein Äquivalent) durch andere Amine, erhält man Kupplungskomponenten der allgemeinen Formel

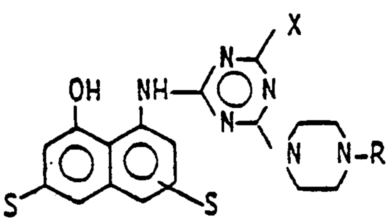

Beachtet werden muß dabei, daß die Umsetzung des naphthalincyanurchloridproduktes mit dem ersten Amin bei 20° bei 50°C und mit dem zweiten Amin bei 50° bis 95°C durchgeführt wird. Sinnvollerweise führt man nach der ersten Aminumsetzung eine Zwischenisolierung durch.

| Bsp. | Naphthalin-disulfonsäure | HX | R |
|---|---|---|---|
| 16.1 | H-Säure | 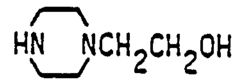 | $-CH_2CH_2NH_2$ |
| 16.2 | " | 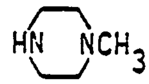 | 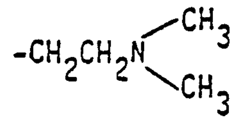 |
| 16.3 | " | 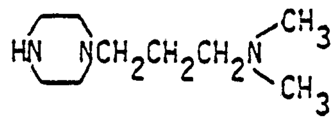 | 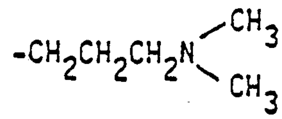 |
| 16.4 | " | " | $-CH_2CH_2NH_2$ |
| 16.5 | " | 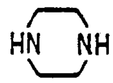 | " |
| 16.6 | " | 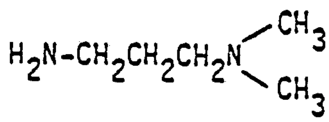 | " |
| 16.7 | " | 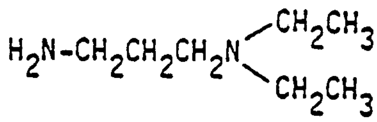 | " |
| 16.8 | " | 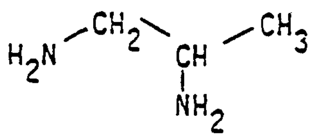 | " |
| 16.9 | H-Säure | 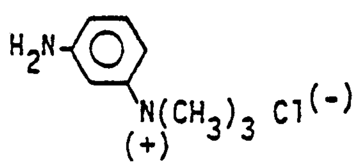 | 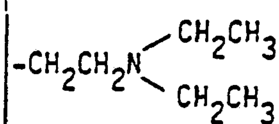 |
| 16.10 | " | 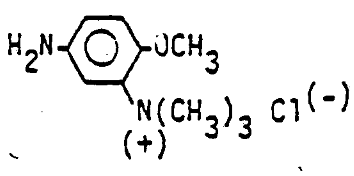 | " |
| 16.11 | " | 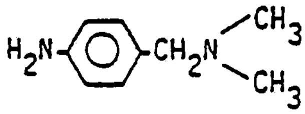 | " |
| 16.12 | " | 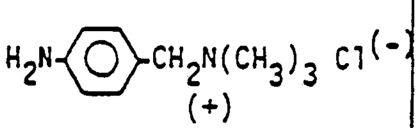 | " |
| 16.13 | K-Säure | 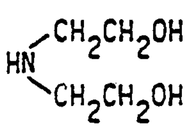 | $-CH_2CH_2NH_2$ |

| Bsp. | Naphthalin-disulfonsäure | HX | R |
|---|---|---|---|
| 16.14 | " | 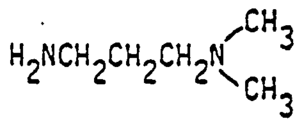 | " |
| 16.15 | " | 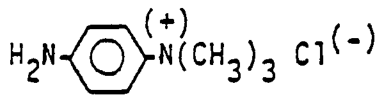 | 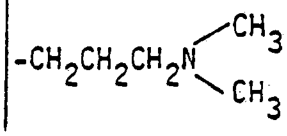 |

Die so erhaltenen Kupplungskomponenten können zu wert-vollen Farbstoffen weiterverarbeitet werden.

Beispiel 17

Analog Beispiel 1 kuppelt man die Diazotierung von 174 g (0,725 Mol) 4-(6-Methylbenzthiazo-2-yl)anilin auf die äquivalente Menge der Kupplungskomponente aus Beispiel 16.3. Man erhält einen Farbstoffe, der Papier in blaustichig violetten Tönen färbt.

Beispiel 18

11,2 g (0,029 Mol) der Diazokomponente der Formel

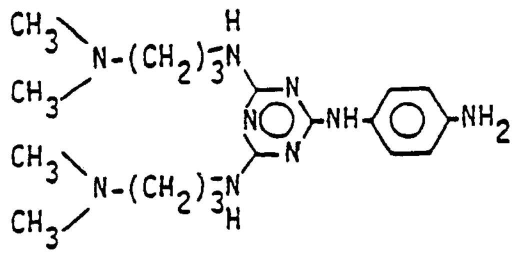

die durch Kondensation von Cyanurchlorid mit 4-Nitroanilin, Eintragen in 3-Dimethylaminopropylamin und anschließende Reduktion hergestellt wurde, werden unter Zugabe von 12 ml 28%iger HCl in 200 ml Wasser gelöst und mit 20,5 ml 10%iger $NaNO_2$-Lösung bei 0—5°C diazotiert. Die Diazotierung wird zu der äquivalenten Menge der Kupplungskomponente 16.13 gegeben und die Kupplung wird bei 5—10°C und pH 6—7 mit festem Natriumhydrogencarbonat durchgeführt. Die ameisensaure Lösung des Farbstoffs färbt Papier in blaustichig violetten Tönen.

**Patentansprüche**

1. Triazinverbindungen der allgemeinen Formel

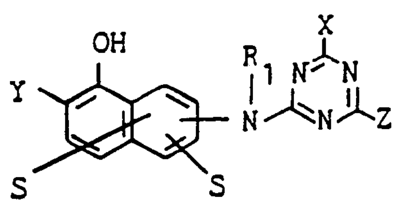

(I)

worin

$R_1$ Wasserstoff oder Alkyl,

S eine Sulfonsäuregruppe oder deren Salz,

X Hydroxy, Alkoxy, Alkyl, Aryl oder eine gegebenenfalls mono- oder di-substituierte Aminogruppe,

Y Wasserstoff oder den Rest eines Azofarbatoffs,

Z ein Rest der Formel

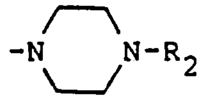 oder 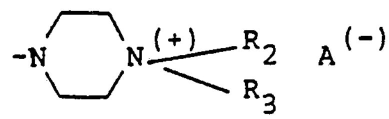,

$R_2$ und $R_3$ unabhängig voneinander Wasserstoff, Alkyl, Alkenyl oder Aralkyl und
$A^{\ominus}$ ein Anion bedeuten,

worin die cyclischen und acyclischen Reste weitere Substituenten tragen können, und worin im Farbstoffmolekül die Summe der basischen und/oder kationischen Gruppen größer als 2 ist, und das Molekül mindestens eine basische oder Kationische gruppe mehr enthält als das Molekül Sulfonsäuregruppen besitzt, mit Ausnahme des Farbstoffe des Bep. 3i der FR—A 2 525 620.

2. Triazinverbindungen der Formel des Anspruchs 1, in der
Y für den Rest

$$D—(N=N—A)_c—N=N—$$

steht, worin
D den Rest einer Diazokomponente der Benzol-, Naphthalin- oder heterocyclischen Reihe,
A den Rest einer Kupplungskomponente der Benzol- oder Naphthalinreihe und
c 0 oder 1 bedeuten,
der Substituenten S in 3,5- oder 3,6-Stellung, der Aminotriazinyl-Rest in 8-Stellung und $R_1$ für Wasserstoff stehen, und S, X und Z die Bedeutung des Anspruchs 1 haben.

3. Triazinverbindungen der Formel des Anspruchs 1, in der
Y und $R_1$ für Wasserstoff,
die Substituenten S in 3,5- oder 3,6-Stellung und der Aminotriazinylrest in 8-Stellung stehen, und S, X und Z die Bedeutung des Anspruchs 1 haben.

4. Triazinverbindungen gemäß Anspruch 1 der Formel

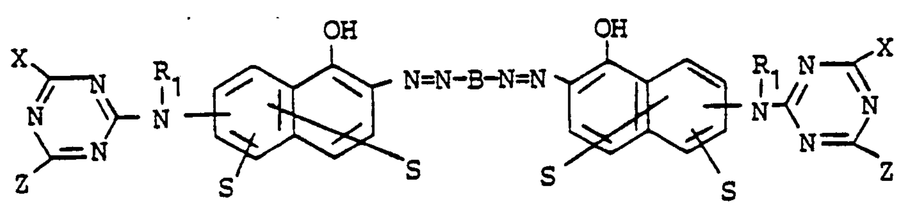

worin
B den Rest einer aromatischen Tetrazokomponente darstellt,
S, X, Z und $R_1$ die Bedeutung des Anspruchs 1 haben,
und worin die Summe der basischen und kationischen Gruppen im Molekül größer als 4, vorzugsweise größer als 5 ist.

5. Triazinverbindungen gemäß Anspruch 1 der Formel

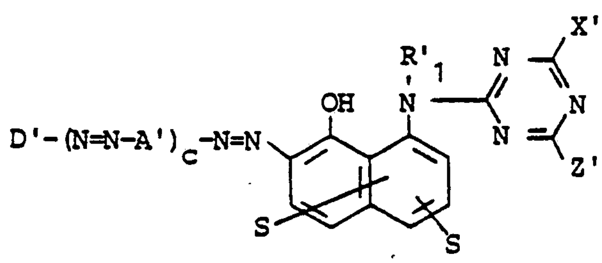

worin
$R_1'$ Wasserstoff oder Methyl,
X' einen Rest der Formel

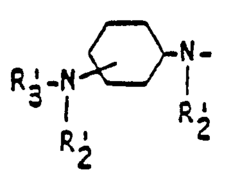 , 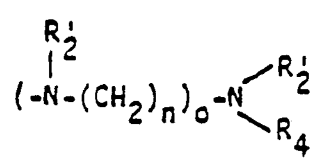

oder

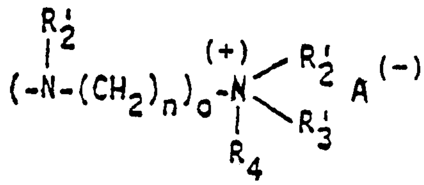

Z' einen Rest der Formel

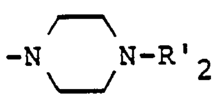 oder 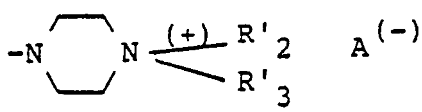

$R_2'$ und $R_3'$ unabhängig voneinander Waserstoff, $C_1$—$C_4$-Alkyl, $C_3$- oder $C_4$-Alkenyl, Benzyl, Phenylethyl, die durch Hydroxy, $C_1$—$C_4$-Alkoxy, Halogen, Cyan, Amino oder Mono- und Di-$C_1$—$C_4$-alkylamino respektive Ammonium und der Benzyl- und Phenylethylrest zusätzlich durch $C_1$- bis $C_4$-Alkyl substituiert sein können,

n 2 oder 3 und zusätzlich 0, wenn o = 1 ist,

o 0, 1 oder 2,

$R_4$ den Rest $R_2'$ und zusätzlich gegebenenfalls durch Halogen, $C_1$—$C_4$-Alkyl, Hydroxy, $C_1$—$C_4$-Alkoxy und/oder den Rest

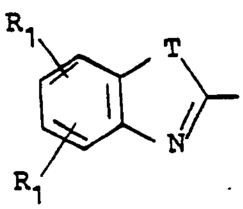

T = S, N-H

substituiertes Phenyl oder Naphthyl, Benzthiazolyl-(2) oder Isobenzthiazolyl-(3) bedeuten, oder

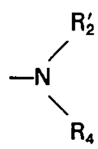

einen Pyrrolidin-, Piperidin- oder Morpholinring oder einen Ring der Formel

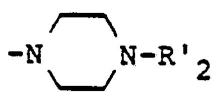 oder 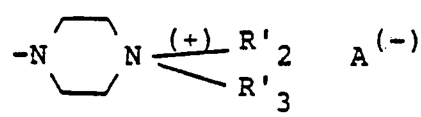

bedeutet,

D' einen Phenyl-, Naphthyl-, Benzthiazolyl- oder Dibenzofuranylrest und

A' einen Phenylen- oder Naphthylenrest bedeuten, wobei

D' und A' durch Halogen, $C_1$—$C_4$-Alklyl, $C_1$—$C_4$-Alkoxy, Acetylamino, Benzoylamino, Phenoxy oder die Gruppen

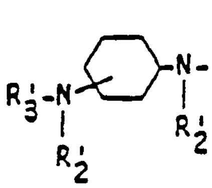 , 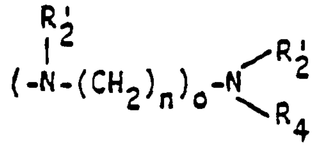 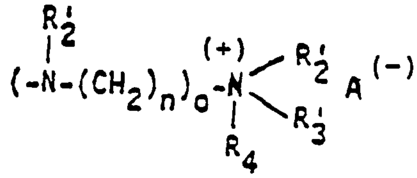 ,

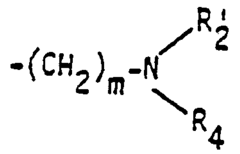,
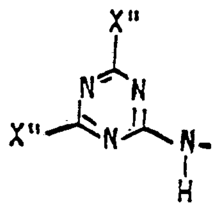,
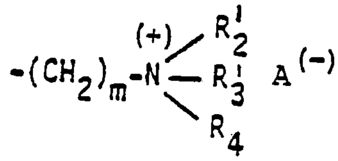

oder eine Sulfonsäuregruppe substituiert sein können,

X″ den Rest X′ und zusätzlich die Reste

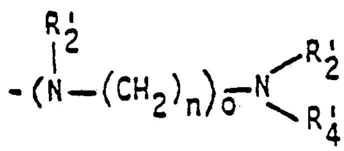 oder 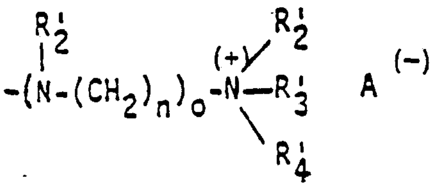

bedeuten, worin

$R_4'$ gegenenfalls durch die Reste

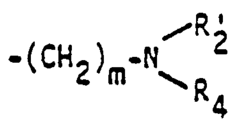 oder 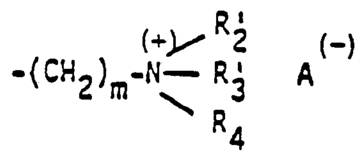

substituiertes Phenyl oder Naphthyl bedeutet,

m und c 0 oder 1 sein können und

S und $A^{\ominus}$ die Bedeutung des Anspruchs 1 haben.

6. Triazinverbindung gemäß Anspruch 1 der Formel

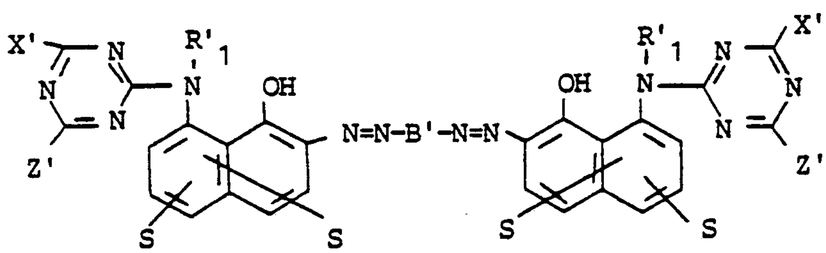

worin

B′ einen Phenylen- oder Naphthylen-Rest oder einen Rest der Formel

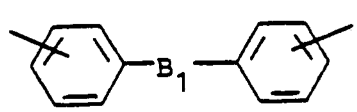

bedeutet, worin

$B_1$ eine direkte Bindung, $-(CH_2)_p-$, —NH—CO—, —O—, $-SO_2-$, —NH—CO—NH—, $-O(CH_2)_p-O-$, $-NH-CO-(CH_2)_p-CO-NH-$, $-CO-NH-(CH_2)_p-NHCO-$ oder

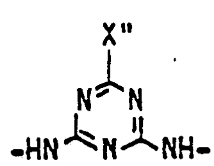

und

p 2 oder 3 bedeuten,

wobei die isocyclischen Ringe durch Halogen, $C_1$—$C_4$-Alkyl, $C_1$—$C_4$-Alkoxy, Acetylamino, Benzoylamino, Phenoxy, die Gruppen

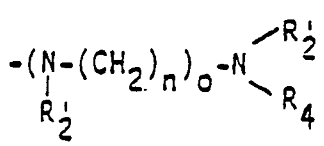 , 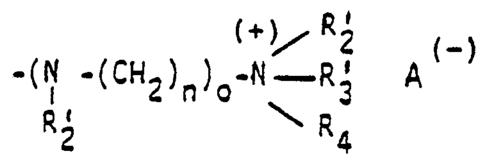

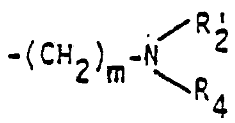 oder 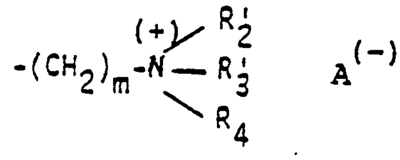

oder die Sulfonsäuregruppe substituiert sein können, und $R_1'$, $R_2'$, $R_3'$, $R_4$, X', X'', m, n und o die in Anspruch 5 angegebene Bedeutung haben.

7. Verfahren zur Herstellung von Triazinverbindungen des Anspruchs 2, dadurch gekennzeichnet, daß man Verbindungen der Formel

$$D—(N=N—A)_c—NH_2$$

diazotiert und mit Verbindungen der Formel

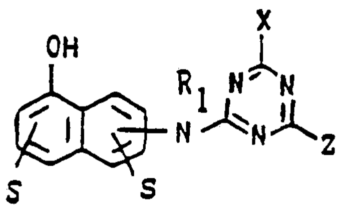

worin die Symbole die in Anspruch 1 angegebene Bedeutung haben, kuppelt.

8. Verfahren zur Herstellung von Triazinverbindungen des Anspruchs 3, dadurch gekennzeichnet, daß man Cyanurhalogenide in beliebiger Reihenfolge mit Verbindungen der Formeln

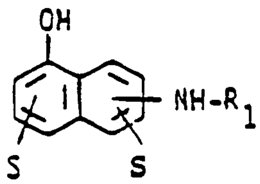 , XH und 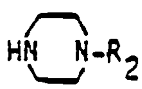 bzw. 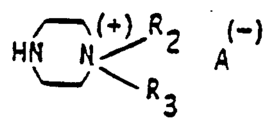

worin die Symbole die in Anspruch 1 angegebene Bedeutung haben, umsetzt.

9. Verfahren zum Färben von natürlichen und synthetischen kationisch anfärbbaren Substraten und Massen, insbesondere Papier, dadurch gekennzeichnet, daß man Farbstoffe des Anspruchs 1 (Y = Rest eines Azofarbstoffs) verwendet.

**Revendications**

1. Composés triaziniques de formule générale

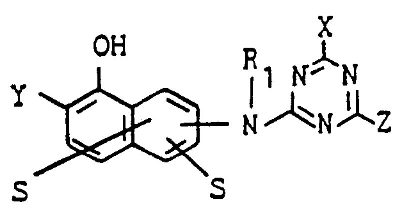

dans laquelle

$R_1$ est l'hydrogène ou un groupe alkyle,

S est un groupe acide sulfonique ou un sel de cet acide,

X est un groupe hydroxy, alkoxy, alkyle, aryle ou un groupe amino éventuellement monosubstitué ou disubstitué,

Y représente l'hydrogène ou le reste d'un colorant azoïque,

Z est un reste de formule

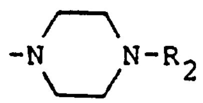 ou 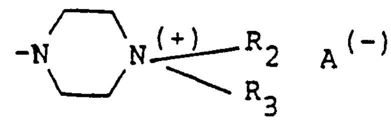 ,

$R_2$ et $R_3$ représentent, indépendamment l'un de l'autre, l'hydrogène, un groupe alkyle, alcényle ou aralkyle et

$A^{\ominus}$ désigne un anion,

les restes cycliques et acycliques pouvant porter d'autres substituants, et dans la molécule du colorant, la somme des groupes basiques et/ou des groupes cationiques étant supérieure à 2 et la molécule contenant au moins un groupe basique ou cationique de plus que la molécule ne possède de groupes acide sulfonique, à l'exception du colorant de l'exemple 31 du document FR—A 2 525 620.

2. Composés triaziniques de formule suivant la revendication 1, dans lesquels

Y représente le reste

$$D—(N=N—A)_c—N=N—$$

où

D est le reste d'un composant diazotable de la série benzénique, naphtalénique ou hétérocyclique,

A est le reste d'un copulant de la série benzénique ou naphtalénique et

*c* a la valeur 0 ou 1,

les substituants S occupent les positiones 3,5 ou 3,6, le reste aminotriazinyle est en position 8 et $R_1$ représente l'hydrogène, et S, X et Z ont la définition donnée dans la revendication 1.

3. Composés triaziniques de formule suivant la revendication 1, dans lesquels

Y et $R_1$ représentent l'hydrogène,

les substituants S sont en positions 3,5 ou 3,6 et le reste aminotriazinyle est en position 8, et S, X et Z ont la définition donnée dans la revendication 1.

4. Composés triaziniques suivant la revendication 1 de formule

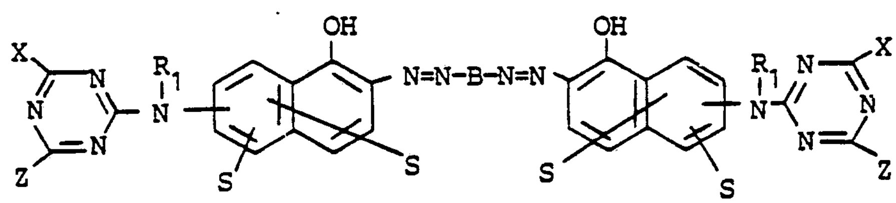

dans laquelle

B est le reste d'un composant tétrazoïque aromatique,

S, X, Z et $R_1$ ont la définition donnée dans la revendication 1,

et la somme des groupes basiques et cationiques dans la molécule est supérieure à 4, de préférence supérieure à 5.

5. Composés triaziniques suivant la revendication 1, de formule

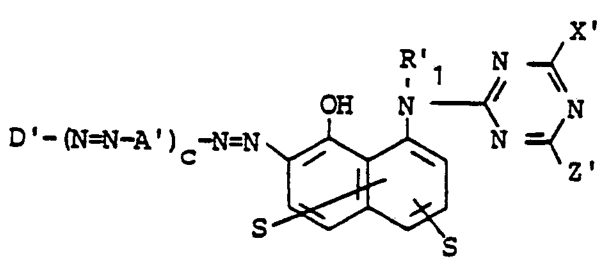

dans laquelle

$R_1'$ est l'hydrogène ou le groupe méthyle,

X' est un reste de formule

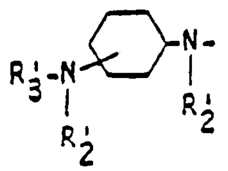 , 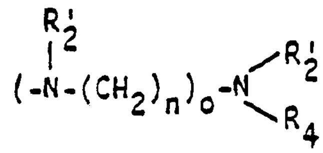

ou

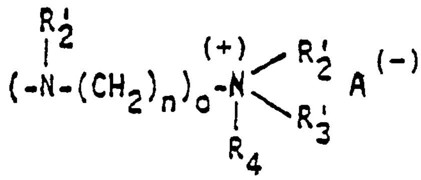

Z' est un reste de formule

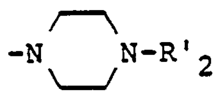 ou 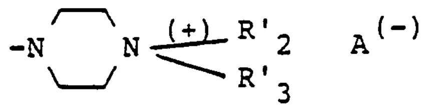

$R'_2$ et $R'_3$ représentent, indépendamment l'un de l'autre, l'hydrogène, un reste alkyle en $C_1$ à $C_4$, un reste alcényle en $C_3$ ou $C_4$, un reste benzyle, phényléthyle, qui peuvent être substitués par un radical hydroxy, alkoxy, en $C_1$ à $C_4$, halogéno, cyano, amino ou mono- ou di-(alkyle en $C_1$ à $C_4$)-amino ou, respectivement, ammonium et le reste benzyle et le reste phényléthyle peuvent en outre être substitués par un radical alkyle en $C_1$ à $C_4$.

*n* a la valeur 2 ou 3 et, en outre, la valeur 0 lorsque *o* est égal à 1,

*o* a la valeur 0, 1 ou 2,

$R_4$ représente le reste $R'_2$ et, en outre, un groupe phényle ou naphtyle éventuellement substitué par un halogène, un reste alkyle en $C_1$ à $C_4$, hydroxy, alkoxy en $C_1$ à $C_4$ et/ou le reste

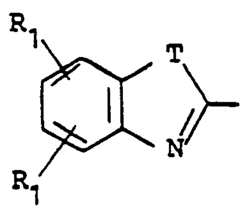 T = S, N-H

un groupe benzothiazolyle-(2) ou isobenzothiazolyle-(3), ou bien

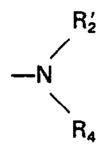

représente un noyau de pyrrolidine, de pipéridine ou de morpholine ou un noyau de formule

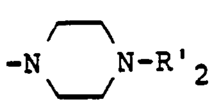 ou 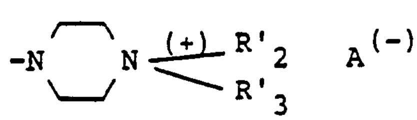

D' est un reste phényle, naphtyle, benzothiazolyle ou dibenzofurannyle et

A' est un reste phénylène ou naphtylène,

D' et A' pouvant être substitués par un halogène, un groupe alkyle en $C_1$ à $C_4$, alkoxy en $C_1$ à $C_4$, acétylamino, benzoylamino, phénoxy ou les groupes

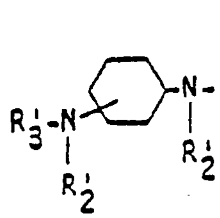

,

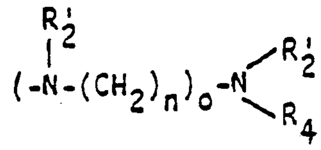

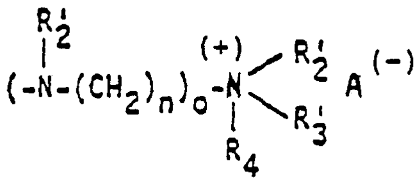

,

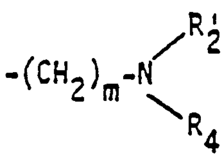

,

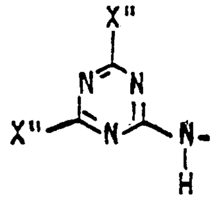

,

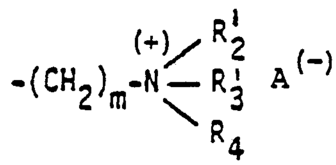

ou par un groupe acide sulfonique,

X'' représente le reste X' et en outre les restes

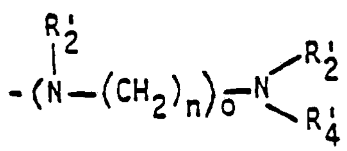

ou

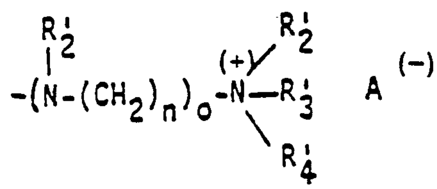

où

$R_4'$ désigne un groupe phényle ou naphtyle éventuellement substitué par les restes

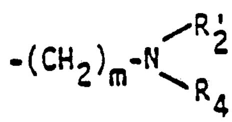

ou

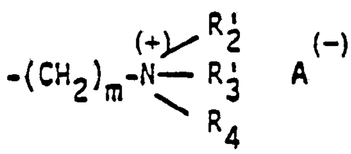

*m* et *c* peuvent avoir la valeur 0 ou 1 et

S et $A^{\ominus}$ ont la définition donnée dans la revendication 1.

6. Composés triaziniques suivant la revendication 1, de formule

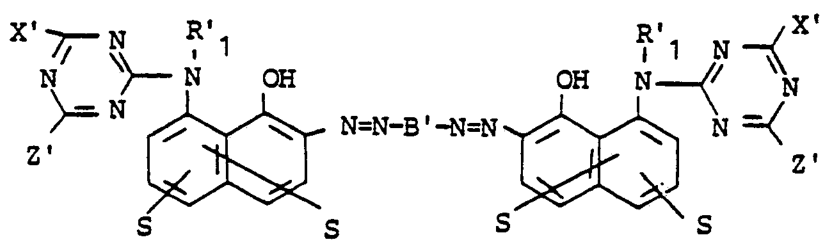

dans laquelle

B' est un reste phénylène ou naphtylène ou un reste de formule

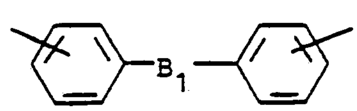

dans laquelle

$B^1$ est une liaison simple, un groupe $-(CH_2)_p-$, $-NH-CO-$, $-O-$, $-SO_2-$, $-NH-CO-NH-$, $-O(CH_2)_p-O-$, $-NH-CO-(CH_2)_p-CO-NH-$, $-CO-NH-(CH_2)_p-NHCO-$ ou

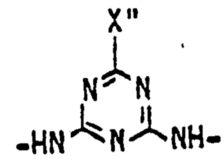

et

*p* a la valeur 2 ou 3,

les noyaux isocycliques pouvant être substitués par un halogène, un groupe alkyle en $C_1$ à $C_4$, alkoxy en $C_1$ à $C_4$, acétylamino, benzoylamino, phénoxy, les groupes

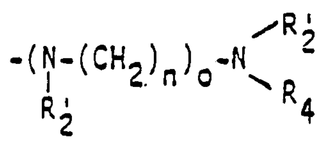 , 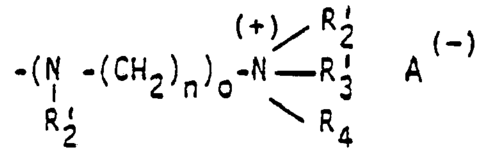

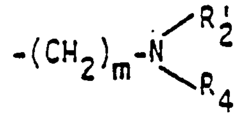 ou 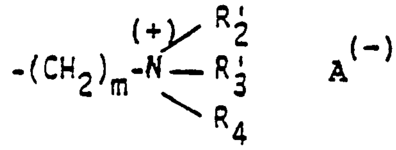

ou le groupe acide sulfonique et $R_1'$, $R_2'$, $R_3'$, $R_4$, X',X'', *m*, *n* et *o* ont la définition indiquée dans la revendication 5.

7. Procédé de production de composés triaziniques suivant la revendication 2, caractérisé en ce qu'on diazote des composés de formule

$$D—(N=N—A)_c—NH_2$$

et on les fait coupler avec des composés de formule

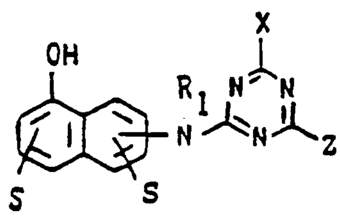

les symboles ayant la définition indiquée dans la revendication 1.

8. Procédé de production de composés triaziniques suivant la revendication 3, caractérisé en ce qu'on fait réagir des halogénures de cyanuryle dans un ordre quelconque avec des composés de formules

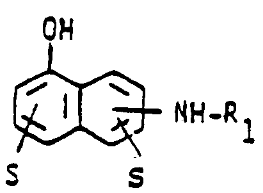 , XH et 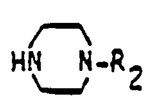 ou 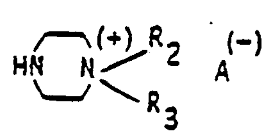

où les symboles ont la définition indiquée dans la revendication 1.

9. Procédé de teinture de matières et de substrats naturels et synthétiques pouvant être teints par un colorant cationique, notamment de papier, caractérisé en ce qu'on utilise des colorants suivant la revendication 1 (Y = reste d'un colorant azoïque).

## Claims

1. Triazine compounds of the general formula

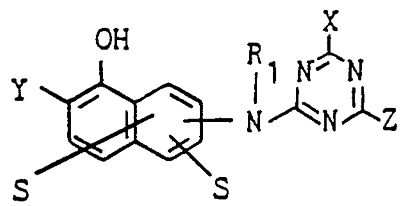

wherein

$R_1$ denotes hydrogen or alkyl,
S denotes a sulphonic acid group or its salts,
X denotes hydroxyl, alkoxy, alkyl, aryl or an optionally mono- or di-substituted amino group,
Y denotes hydrogen or the radical of an azo dyestuff,
Z denotes a radical of the formula

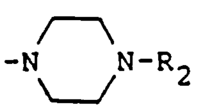 oder 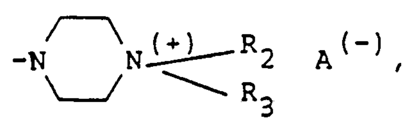

$R_2$ and $R_3$ independently of each other denote hydrogen, alkyl, alkenyl or aralkyl, and
$A^{\ominus}$ denotes an anion,

wherein the cyclic and acyclic radicals can carry further substituents, and wherein, in the dyestuff molecule, the total number of the basic and/or cationic groups is greater than 2 and the molecule contains at least one basic or cationic group more than the molecule has sulphonic acid groups, with the exception of the dyestuff of Example 31 of FR—A—2 525 620.

2. Triazine compounds of the formula of Claim 1, in which
Y represents the radical

$$D-(N=N-A)_c-N=N-$$

wherein

D denotes the radical of a diazo component of the benzene, naphthalene or heterocyclic series,
A denotes the radical of a coupling component of the benzene or naphthalene series and
c denotes 0 or 1,

the substituents S are in the 3,5- or 3,6-position, the aminotriazinyl radical is in the 8-position and $R_1$ represents hydrogen, and S, X and Z have the meanings of Claim 1.

3. Triazine compounds of the formula of Claim 1, in which
Y and $R_1$ represent hydrogen, and
the substituents S are in the 3,5- or 3,6-position and the aminotriazinyl radical is in the 8-position, and S, X and Z have the meanings of Claim 1.

4. Triazine compounds according to Claim 1, of the formula

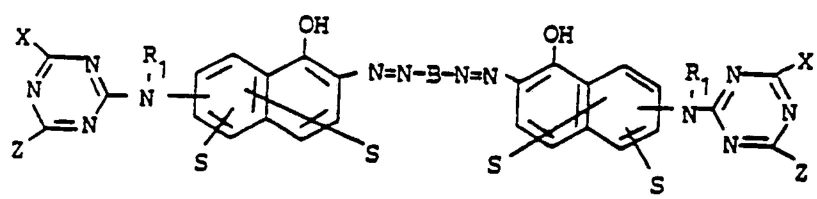

wherein

B represents the radical of an aromatic tetrazo component,
S, X, Z and $R_1$ have the meanings of Claim 1,

and wherein the total number of basic and cationic groups in the molecule is greater than 4, preferably greater than 5.

5. Triazine compounds according to Claim 1, of the formula

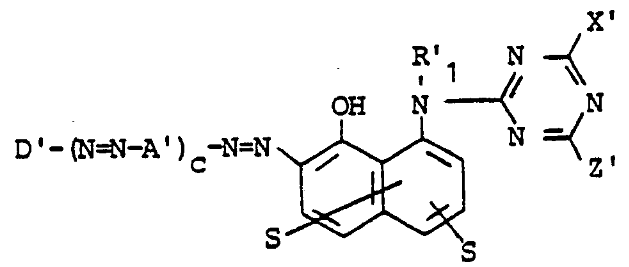

wherein

X′ denotes a radical of the formula

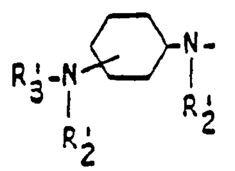 , 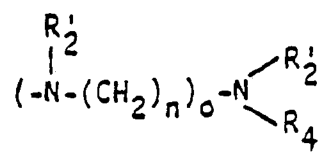

or

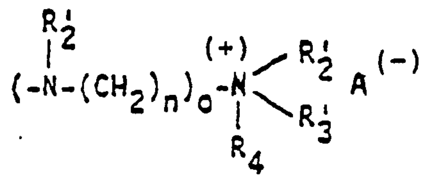

Z′ denotes a radical of the formula

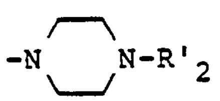 or 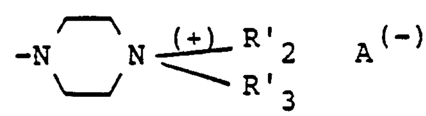

$R_2'$ and $R_3'$ independently of each other denote hydrogen, $C_1$—$C_4$-alkyl, $C_3$- or $C_4$-alkenyl, benzyl or phenylethyl either of which can be substituted by hydroxyl, $C_1$—$C_4$-alkoxy, halogen, cyano, amino or mono- and di-$C_1$—$C_4$-alkylamino or ammonium and, in the case of the benzyl and phenylethyl radicals, also by $C_1$- to $C_4$-alkyl,

n denotes 2 or 3 and additionally 0 when o = 1, o = 0, 1 or 2,

$R_4$ denotes the radical $R_2'$ and also optionally halogen-, $C_1$—$C_4$-alkyl-, hydroxyl-, $C_1$—$C_4$-alkoxy- and/or

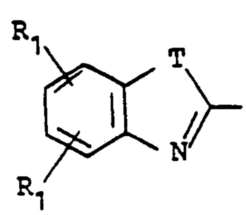 T = S, N-H

substituted phenyl or naphthyl, benzothiazol-2-yl or isobenzothiazol-3-yl, or

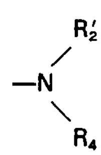

denotes a pyrrolidine, piperidine or morpholine ring or a ring of the formula

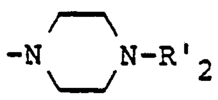 or 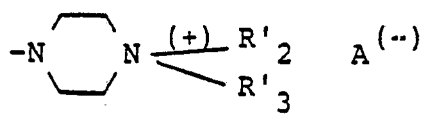

D′ denotes a phenyl, naphthyl, benzothiazolyl or dibenzofuranyl radical, and

A′ denotes a phenylene or naphthylene radical where D′ and A′ can be substituted by halogen, $C_1$—$C_4$-alkyl, $C_1$—$C_4$-alkoxy, acetylamino, benzoylamino, phenoxy, the groups

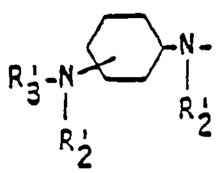 , 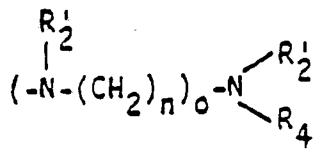

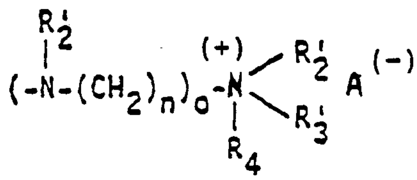 ,

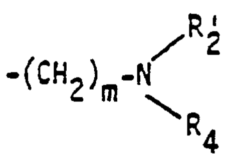 , 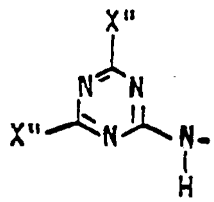 , 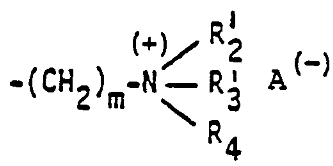

or a sulphonic acid group,

X″ denotes the radical X′ and also the radicals

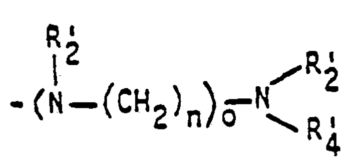 or 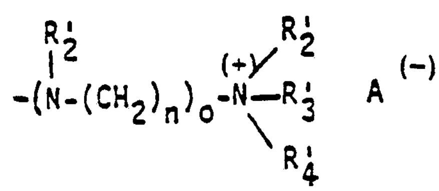

wherein

$R_4'$ denotes naphthyl or phenyl which can optionally be substituted by the radicals

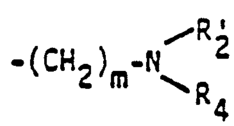 or 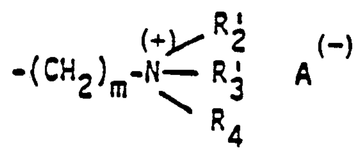

m and c can be 0 or 1, and

S and $A^{\ominus}$ have the meanings of Claim 1.

6. Triazine compounds according to Claim 1, of the formula

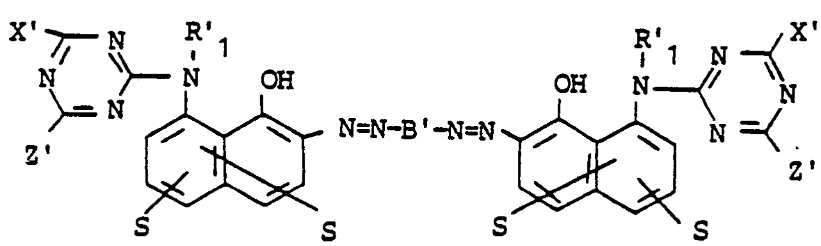

wherein

B′ denotes a phenylene or naphthylene radical or a radical of the formula

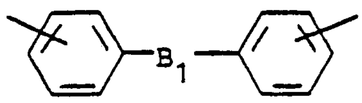

wherein

$B_1$ denotes a direct bond, $—(CH_2)_p—$, —NH—CO—, —O—, $—SO_2—$, —NH—CO—NH—, $—O(CH_2)_p—O—$, $—NH—CO—(CH_2)_p—CO—NH—$, $—CO—NH—(CH_2)_p—NHCO—$ or

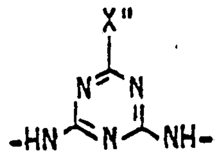

and

p denotes 2 or 3,

wherein the isocyclic rings can be substituted by halogen, $C_1—C_4$-alkyl, $C_1—C_4$-alkoxy, acetylamino, benzoylamino, phenoxy, the groups

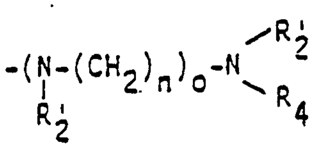 , 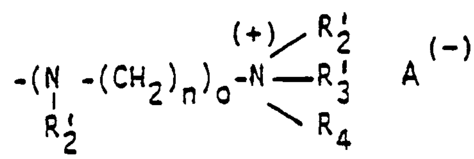

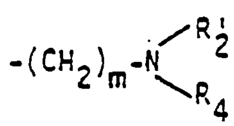 or 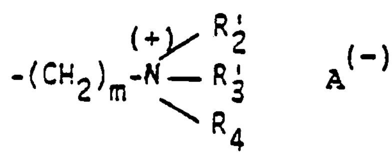

or the sulphonic acid group, and $R_1'$, $R_2'$, $R_3'$, $R_4$, X', X'', m, n and o have the meanings given in Claim 5.

7. Process for preparing triazine compounds of Claim 2, characterised in that compounds of the formula

$$D—(N{=}N—A)_c—NH_2$$

are diazotised and coupled with compounds of the formula

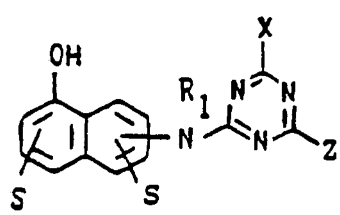

wherein the symbols have the meanings given in Claim 1.

8. Process for preparing triazine compounds of Claim 3, characterised in that cyanuric halides are reacted in any order with compounds of the formulae

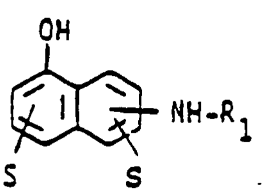 , XH and 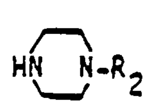 or 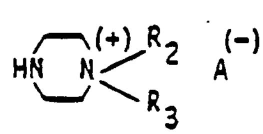

wherein the symbols have the meanings given in Claim 1.

9. Process for dyeing natural and synthetic cationically dyeable substrates and compositions, particularly paper, characterised in that dyestuffs of Claim 1 (Y = the radical of an azo dyestuff) are used.